# EUROPEAN PATENT APPLICATION

(11) **EP 0 647 719 A1**
(43) Date of publication of application: **12.04.1995**
(21) Application number: 93906812.8
(22) Date of filing: 22.03.1993
(51) Int. Cl.: C12Q 1/68, C12N 15/10

(54) **SOLID-PHASE ANCHOR CARRYING NUCLEIC ACID AND METHOD OF ADJUSTING THE SAME**

(71) Applicant: KABUSHIKI KAISHA NIPPON GENE, Tokyo 160 (JP)
(72) Inventor: YONEDA, Yukou, K.K. Nippon Gene, Toyama-shi, Toyama 930 (JP)
(74) Representative: Schrimpf, Robert
(86) International application number: JP9300348
(87) International publication number: WO9421819

(57) **Abstract**

A solid-phase anchor carrying nucleic acids which can amplify the amount of nucleic acid probes on a solid-phase support and is satisfactorily usable in the separation and purification of DNA-binding proteins on a practical scale and in gene cloning; and a method of adjusting the anchor. The anchor contains a solid-phase support provided with an amplifier group having a number of branching ends, each of which has a binding group which can be bound to a functional group positioned at one end of a nucleic acid fragment to be bound to a predetermined nucleic acid probe. By appropriately selecting the amplifier group, it is possible to form hundreds to thousands of binding groups at one binding site of the solid-phase support and thus mount a number of nucleic acid probes.

## Description

### TECHNICAL FIELD

The present invention relates to an anchor for immobilizing nucleic acids, capable of immobilizing or deimmobilizing a vast amount of DNA required for the separation and purification of DNA binding proteins and for DNA cloning, and to a method for preparing the same.

### BACKGROUND ART

cDNA cloning having been frequently carried out in recent years requires preliminary extraction and purification of mRNA. For such purpose, a single stranded oligo-dT immobilized onto a solid phase has been available currently. In such case, mRNA should be once separated and purified prior to subsequent cDNA synthetic process. Because the separation and purification process involves more or less mRNA decomposition with a resultant lower recovery, the process still remains within the range of conventional plate screening.

For isolation of a protein binding to a double stranded DNA having a specific nucleotide sequence or for detecting or separating an objective DNA by using a nucleic acid probe, a highly efficient and simple process of immobilizing a double stranded DNA onto a solid carrier is a very useful technique. However, such technical process has not yet been known.

Furthermore, cDNA synthesis on the basis of mRNA isolated by using oligo-dT attached onto a solid carrier has frequently been done in recent years. In this case, mRNA is purified after its preliminary dissociation from the immobilized oligo-dT. Then, the thus purified mRNA is used for cDNA synthesis. It should be expected that if such reaction is done in a solid state, the efficiency and yield of the reaction may be distinctively elevated, but conventional oligo-dT is problematic because a double stranded cDNA as the final product cannot be separated readily from such solid carrier.

Alternatively, it is known for example in Japanese Patent Application No. H3-147800 an anchor for immobilizing a nucleic acid, capable of enzymatically binding the nucleic acid to a solid carrier along with a method for preparing the same.

When cloning is done by a solid phase method, it is evident that nucleic acid probes as well as desired DNA fragments are used as in the form of double stranded DNAs in most cases and that nucleic acid probes on a solid phase are attached to the solid carrier while the desired DNA fragments are suspended in solution.

In the hybridization of a desired DNA fragment with a nucleic acid probe on a solid carrier, therefore, the probability that a single stranded DNA from the desired DNA fragment hybridizes with the nucleic acid probe (single strand) on the solid carrier is far lower than the probability of the occurrence of the annealing of the single stranded DNA to its complementary DNA prior to the hybridization.

Thus, the amount of the DNA fragment to be hybridized onto the nucleic acid probe on the solid carrier is much less, which is almost impossible to be detected by gel electrophoresis unless a detector with an extremely high sensitivity is used. Hence, the technique disclosed in the Japanese Patent Application No. H3-147800 is hardly applicable, in practical sense, to the separation and purification of DNA binding proteins or to DNA cloning.

### Disclosure of the Invention

The objective of the present invention resides in providing an anchor for immobilizing nucleic acids, capable of amplifying the amount of a nucleic acid probe on a solid carrier at such a higher efficiency and a higher yield that the amplified nucleic acid probe can be used satisfactorily for the separation and purification of DNA binding proteins or for DNA cloning at a practical scale, and also resides in providing a method for preparing the same.

According to the essential embodiment of the present invention, the anchor for immobilizing nucleic acids includes a solid carrier; the solid carrier is provided with amplifying groups each having a plurality of branched ends; and at a plurality of the branched ends are individually formed binding groups each capable of binding to a functional group arranged at one end of a DNA fragment which is to bind to a preliminarily determined nucleic acid probe.

According to a preferable embodiment of the present invention, each amplifying group is composed of a polymer containing a binding group capable of binding to such functional group within the monomer thereof.

According to another preferable embodiment of the present invention, the amplifying group is composed of a polymer selected from a group consisting of polypeptide compounds, cellulose compounds, polyvinyl alcohol compounds, acrylic compounds, or vinyl compounds.

According to still another preferable embodiment of the present invention, the amplifying group is composed of poly-lysine, and the DNA fragment binds to an end branched from the NH₂ groups of the poly-lysine.

For forming binding groups each capable of binding to a functional group arranged at one end of a DNA fragment which is to bind to a preliminarily determined nucleic acid probe, the method for preparing an anchor for immobilizing nucleic acids in accordance with one embodiment of the present invention comprises a first process of activating a solid carrier and a second process of arranging amplifying groups, each amplifying group having a plurality of branched ends to form the binding groups at the activated sites of the solid carrier.

According to a preferable embodiment of the present invention, the second process comprises arranging a polymer having binding groups capable of binding to the functional groups.

According to another preferable embodiment of the present invention, the second process comprises arranging as such amplifying group a polymer selected from a group consisting of polypeptide compounds, cellulose compounds, polyvinyl alcohol compounds, acrylic compounds or vinyl compounds.

According to still another embodiment of the present invention, the second process comprises arranging poly-lysine as the amplifying groups wherein the binding groups are formed at the ends branched from the NH₂ groups of the poly-lysine.

In the anchor for immobilizing nucleic acids in accordance with the present invention, one or more amplifying groups are attached to a solid carrier, and at a plurality of the branched ends of the amplifying groups are individually formed binding groups; and because the binding groups each can bind to a functional group of a DNA fragment which is selected for the binding with a desired nucleic acid probe, a vast number of such binding groups as much as several hundreds to several thousands may be formed on one solid carrier by selecting a variety of types of amplifying groups. Therefore, a great number of nucleic acid probes can be arranged on the anchor.

The anchor for immobilizing nucleic acids in accordance with the present invention can immobilize any double stranded DNA, any single stranded DNA and any RNA probe, readily at a greater amount, if the type of a DNA fragment or the type of a restriction enzyme in the anchor is appropriately selected as described hereinbelow.

When a desired DNA is selected through hybridization for example among double stranded DNAs, therefore, the amount of the DNA in hybridization to the desired nucleic acid probe and immobilized on a solid phase is relatively more, which is sufficient for detection for example by routine agarose gel electrophoresis. Consequently, the anchor for immobilizing nucleic acids in accordance with the present invention is satisfactorily applicable to a practical scale of the separation and purification of DNA binding proteins or to DNA cloning.

On a solid phase, use may be made of any amplifying group, having within the molecule a plurality of binding groups each capable of binding to a functional group. Depending on the functional group selected from a variety of functional groups as the functional group at one end of a DNA fragment so that the fragment may bind to a desired nucleic acid probe, use may be made of any appropriate binding group from various binding groups such as OH group, NH₂ group, COOH group, CONH₂ group and OCH₃ group as the binding groups to be formed at the branched ends of the amplifying groups.

Specifically, each amplifying group comprises a polymer composed of a monomer having a binding group capable of binding to such functional group. For more specific illustration, the polymer may be selected from a group consisting of polypeptide compounds having NH₂ branched group within the molecule of polyglutamic acid, poly-lysine, sericin, fobroin, keratin, collagen and the like; cellulose having branched groups such as OH group, NH₂ group and COOH group within the molecule of carboxymethylcellulose, alginic acid, chitosan and the like; polyvinyl alcohol compounds such as polyvinyl alcohol, ethylene vinyl acroyl copolymer and the like; acrylic compounds such as polymethacrylate hydroxyethyl, polyacrylate, polyacrylic amide; and vinyl compounds such as poly-N-vinylpyrrolidone, polyvinyl methyl ether and the like.

For the anchor of the present invention, any DNA fragment capable of binding to the nucleic acid probe preliminarily determined depending on the objective may be useful, if the fragment has a functional group at one end, specifically a functional group readily binding to a binding group on a solid phase without causing the denaturation of a nucleic acid when the solid carrier and the nucleic acid bind together.

The method for preparing the anchor for immobilizing nucleic acids in accordance with the present invention comprises activating a solid carrier thereby forming activated sites for the binding of amplifying groups onto the solid carrier, binding and arranging the amplifying groups at the activated sites, and forming individual binding groups at a plurality of the branched ends of the amplifying groups. The anchor for immobilizing nucleic acids, prepared by such simple process, is contact to a DNA fragment to bind to the nucleic acid probe preliminarily determined depending on the subject such as the separation and purification of DNA binding proteins or DNA cloning. The binding group is prepared for its ready binding to a functional group formed at one end of the selected DNA fragment. Almost any DNA fragment capable of binding to a desired nucleic acid probe may be used in the anchor for immobilizing nucleic acids in accordance with the present invention. Furthermore, the anchor for immobilizing nucleic acids in accordance with the present invention may attach a DNA fragment preliminarily bound to a desired nucleic acid probe to the binding group thereof.

Specifically, the method for immobilizing nucleic acids in accordance with the present invention varies in details depending on the selected immobilizing carrier, the formation of activated sites, the types of amplifying groups, binding groups and functional groups as the subjects (ie. nucleic acids).

According to one specific embodiment of the present invention, poly-lysine is arranged on a solid carrier as an amplifying group while the DNA fragment binds to a branched end from the NH₂ groups of the polylysine. In such case, non-porous resin carrier in a form of beads may be used as such solid carrier. The solid carrier is subjected to the activation process via bis-oxirane (1,4-butanediol diglycidyl ether; manufactured by Aldrich Chemical, Co. Ltd.), so that the OH group on the surface of the solid carrier is activated. As the amplifying group, poly-lysine binds to and is arranged on the activated OH group. The polylysine has a plurality of NH₂ groups at the branched ends thereof, and at the individual ends of these NH₂ groups is arranged 2-iminothioran, with the result that the SH binding group is formed at each end. As the functional group binding to the SH group, the maleimide group of sulfosuccinimidyl 4-(p-maleimide phenyl)-butyrate may be used. By binding sulfosuccinimidyl 4-(p-maleimide phenyl)-butyrate to the NH₂ group of the 5' terminus of a DNA fragment which is required to bind to a nucleic acid probe so as to make the maleimide group function as the functional group, the DNA fragment should bind to the SH binding group of the solid carrier at a higher probability.

If the DNA fragment which is required to bind to the nucleic acid probe has a cleavage site by a specific restriction enzyme, the DNA fragment can be cleaved with the corresponding restriction enzyme, thereby generating terminal structures. Then, a desired double stranded DNA or a desired single stranded DNA probe, having a corresponding terminus to the above terminal structures, may be ligated via an enzyme such as ligase. Thus, the immobilized nucleic acid probe may be separated readily from non-immobilized substances, with the result that a higher yield and a higher efficiency may be achieved at the process.

Still furthermore, if poly-dT sequence is arranged at the other end of the DNA fragment, a cDNA library may be prepared directly via the use of an enzyme such as a reverse transcriptase, which is then readily used as a nucleic acid probe because the DNA fragment readily binds to the poly A chain of mRNA. The prepared cDNA library may readily be separated from non-immobilized substances, and therefore, the library may readily be inserted into a vector and the like.

Additionally, the binding of such functional group with the binding group may be done by diazo coupling method for binding a functional group to a solid carrier; a coupling method via carbodiimide; a method for directly coupling with bis-oxirane and the like. In other words, the binding may be at any mode which can readily bind a functional group to a binding group without denaturing a nucleic acid when the solid carrier and the nucleic acid bind together completely.

Several preferable embodiments of the present invention will now be described with reference to drawings, but these embodiments are simply illustrated so as to promote understanding of the characteristics and advantages of the present invention. Herein, the technical scope of the present invention is not limited to these embodiments.

### BRIEF DESCRIPTION OF THE INVENTION

Fig.1 is an explanatory view schematically depicting the poly-lysine type-anchor for immobilizing nucleic acids in accordance with one example of the present invention;
Fig.2 is an explanatory view depicting one example of the nucleotide sequence of a DNA fragment;
Fig.3 is an explanatory view depicting the reaction state of a solid carrier during the preparation procedure;
Fig.4 is an explanatory view depicting the reaction state of a DNA fragment during the preparation procedure;
Fig.5 is an explanatory view depicting the reaction state of the solid carrier with the DNA fragment; and
Fig.6 is a figure depicting the results of the agarose gel electrophoretic analysis of the binding of a DNA fragment via the anchor for immobilizing nucleic acids in accordance with one example of the present invention.

### BEST EMBODIMENTS FOR CARRYING OUT THE INVENTION

### Example 1

### Schematic illustration of poly-lysine type-anchor for immobilizing nucleic acids

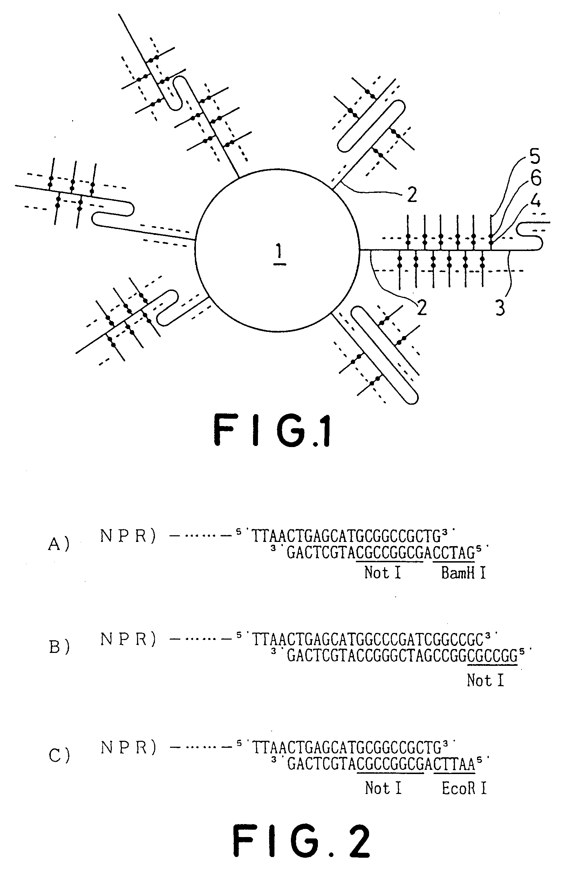

Fig.1 is an explanatory view schematically depicting the poly-lysine type-anchor for immobilizing nucleic acids in accordance with one example of the present invention. The poly-lysine type-anchor for immobilizing nucleic acids as shown in the figure has resin-made solid carrier (1) of NPR (Non-Porous Resin) in a form of beads, and through the activation process with bis-oxirane under alkali conditions, the OH group on the surface of the solid carrier is activated to form activated site (2). To the activated site (2) is ligated amplifying group (3) comprising poly-lysine, and at the individual plural branched ends of the NH₂ groups of the poly-lysine is arranged 2-iminothioran, to form SH binding groups (4). Prior to use, to the binding groups (4) are attached functional groups (6) each at 5' terminus of DNA fragment (5) which is to bind to an objective nucleic acid probe preliminarily determined. In the present example, the functional group (6) is composed of a maleimide group produced by binding sulfosuccinimidyl 4-(p-maleimide phenyl)-butyrate to the NH₂ group at the terminus of the DNA fragment (5).

Because the amplifying group (3) composed of poly-lysine has a great number of branched ends, a great number of binding groups (4) as many as several hundreds to several thousands should be formed to one activated site (2) of the solid carrier. Therefore, the anchor for immobilizing nucleic acids of the present example can mount a vast number of nucleic acid probes; when a desired DNA is selected through hybridization among double stranded DNA fragments, for example, the amount of the desired DNA in hybridization with the solid carrier is sufficient enough for detection by routine agarose gel electrophoresis, which is satisfactorily used for the separation and purification of DNA binding proteins or for DNA cloning at a practical scale.

As DNA fragment (5) which should bind to a nucleic acid probe, use may be made of a variety of DNA fragments. Fig.2 depicts the examples of the nucleotide sequences of DNA fragments usable as the DNA fragment (5) of Fig.1, wherein sequences A, B and C have restriction enzyme cleavage sites (BamH I, Not I, and Ec oR I), into which a desired nucleic acid probe is inserted.

### Example 2

### Preparation of poly-lysine type anchor for immobilizing nucleic acids

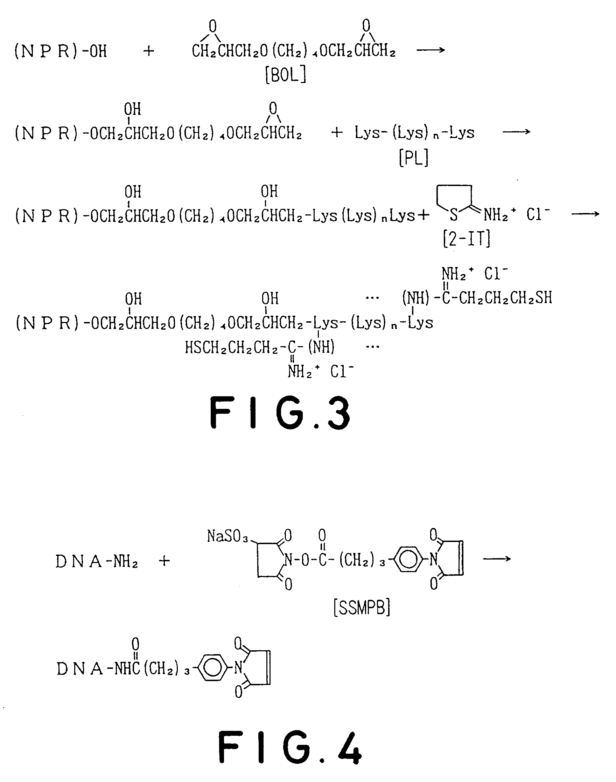

One example of the method for preparing a poly-lysine type anchor for immobilizing nucleic acids will now be described below. Fig.3 is an explanatory view depicting the reaction state of the solid carrier during the preparation procedure. As shown in the figure, the preparation procedure followed the individual processes described below.
(1) Solid carrier NPR (Non Porus Resin, manufactured by Toso, Co. Ltd.; 1 ml) was centrifuged and washed in solution A (20 % DMSO, 0.25N NaOH).
(2) Solution A and bis-oxirane (BOL; 5 ml) reacted with the solid carrier NPR under stirring with a rotator at room temperature for 12 hours. Subsequently, the resulting product was centrifuged and washed in the solution A (× 3), 20 % DMSO (× 2) and distilled water (× 1) to recover activated NPR.
(3) The activated NPR was suspended in poly-lysine (PL of an average molecular weight of 100,00, manufactured by Wako Chemical, Co. Ltd.; 20 mg) dissolved in 0.5 M carbonate buffer solution, pH 10 (40 ml) for reaction under stirring with a rotator at room temperature for 24 hours. Then, the reaction product was washed in 2M sodium chloride solution (× 2), 20 % DMSO (× 2), and distilled water (several times) until no amino group was detected in the supernatant. Thus, poly-lysinyl NPR was recovered.
(4) The poly-lysinyl NPR was suspended in solution B (0.1M phosphate buffer solution, pH 7.4 containing 1mM EDTA), followed by addition of 2-iminothioran (2-IT; manufactured by Sigma, Co. Ltd.; 10 mg) for reaction under stirring with a rotator at room temperature for one hour to form binding groups composed of maleimide group at the individual branched ends of the poly-lysinyl NPR.
   Fig.4 depicts the reaction state of a DNA fragment during the preparation procedure. As shown in the figure, the preparation procedure was done following the processes described below.
(5) A DNA fragment inserted with a preliminarily determined nucleic acid probe reacted with a 10-fold excess of sulfosuccinimidyl 4-(p-maleimide phenyl)-butyrate (SSMPB) at 40°C for 30 minutes, and then, unreactive SSMPB was removed to recover a DNA fragment with an activated functional group.

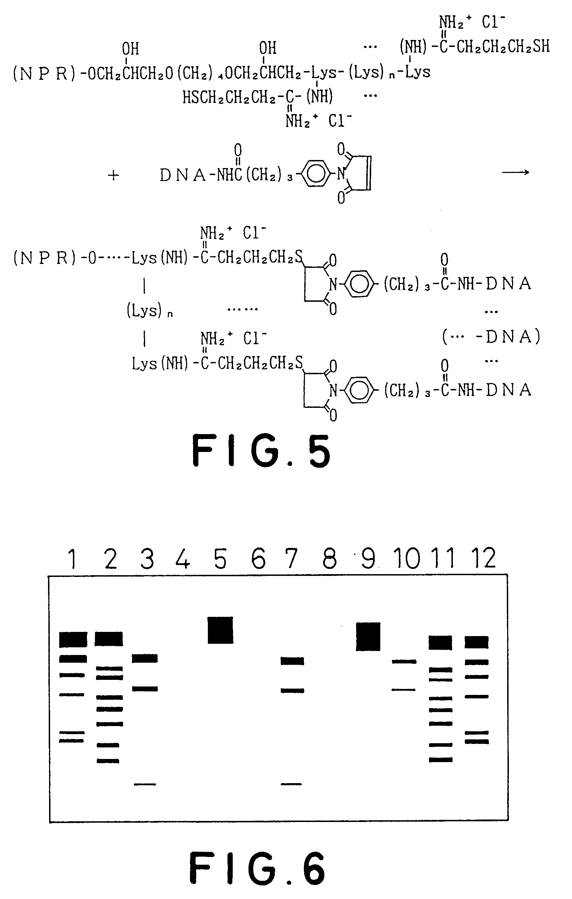

   Fig.5 is an explanatory view depicting the reaction state of the solid carrier with the DNA fragment. As shown in the figure, the preparation procedure was done following the individual processes described below.
(6) The poly-lysinyl NPR from the process (4) was centrifuged and washed in the solution B (× 3) prior to resuspension in the solution B (40 ml). Then, about 0.5 mg of the activated DNA fragment from the process (5) was added to the suspension for reaction under agitation with a rotator at room temperature for four hours or more.
(7) The mixture after completion of the reaction at the process (6) was centrifuged and washed in the solution B (× 3) and 1 × SSC (× 2) prior to addition of the complementary chain (0.75 mg) for thermal treatment at 95°C for 10 minutes. Then, hybridization was carried out at 68°C for one hour. Subsequently, the resulting product was gradually cooled down to room temperature prior to centrifugation and washing in 1 × SSC several times. A part of the resulting product was thermally treated for the quantitative analysis of the binding.

As has been described above, the number of the binding groups in arrangement was relatively amplified. In other words, poly-lysine as the amplifying group bound to the solid carrier, and at each of a plurality of NH₂ groups in the monomer of the poly-lysine is arranged 2-iminothioran which is to function as SH group, and the SH group is used as the amplified binding group.

### Example 3

### Attachment of DNA fragment to anchor for immobilizing nucleic acids and recovery of the DNA fragment

In the present example, an anchor for immobilizing nucleic acids, ie. Not I (solid carrier bound with an anchor DNA having a terminus capable of ligating Not I digested DNA fragment), was prepared by the method shown in Example 2,.

The immobilizing anchor Not I (5 µℓ) was washed twice in 1 × T4 DNA ligase reaction solution containing 0.04 % BSA, and then, to the reaction solution (100 µℓ) was added a DNA produced from digestion of plasmid B315 (16,769 bp in its entire length; produced by cloning a part of Ad2 virus DNA into pBR322) for suspension with the solid carrier.
Furthermore, T4 DNA ligase (referred to as "T4 ligase" hereinafter;
6,000 units) was added to the suspension, for reaction under stirring with a rotator at 15 °C for four hours. A part of the reaction product was centrifuged at 5,000 rpm for 2 minutes, to recover the supernatant and precipitate.

The supernatant (about 100 µℓ) was stored until the time of electrophoretic analysis. The precipitate was gently washed in 1 × TES solution (10mM Tris.HCl, 1mM EDTA and 50mM NaCl) (× 1) and 1 × Not I reaction solution containing 0.04 % BSA (× 2), and then suspended in 1 × Not I reaction solution (10 µℓ). Further,Not I (100 units) was added to the resulting solution, for reaction under stirring with a rotator at 37°C for three hours. The reaction solution was centrifuged at 5,000 rpm for 2 minutes, and together with the preliminarily stocked supernatant, the present supernatant was subjected to the analysis by 0.8 % agarose gel electrophoresis to quantitatively determine the binding of the Not I DNA fragment.

Fig.6 shows the results of the analysis by agarose gel electrophoresis. In the figure, lane 1 represents λDNA/Hind III digested molecular weight marker; lane 2, λDNA/Sty I digested molecular weight marker; lane 3, NPR-Not I anchor (Comparative Example; with no addition of T4 ligase); lane 4, NPR-Not I anchor (Comparative Example; with no addition of T4 ligase; digestion with Not I); lane 5, NPR-Not I anchor (Comparative Example; with T4 ligase added ); lane 6, NPR-NotI anchor (Comparative Example; with T4 ligase added; digestion with Not I); lane 7, poly-lysinyl NPR-Not I anchor (Example; with no addition of T4 ligase); lane 8, poly-lysinyl NPR-Not I anchor (Example; with no addition of T4 ligase; digestion with Not I); lane 9, poly-lysinyl NPR-Not I anchor (Example; with addition of T4 ligase); lane 10, poly-lysinyl NPR-Not I anchor (Example; with addition of T4 ligase;
digestion with Not I); lane 11, the same as lane 2; and lane 12, the same as lane 1.

As shown in Fig.6, no DNA fragment was recovered or detected in the Comparative Examples with no amplification with poly-lysine, but DNA fragment was recovered only from a sample of NPR treated with poly-lysine and through T4 ligase reaction (lane 10). The results shown in lane 10 indicate that DNA (about 0.8µg /5µℓ resin) in binding to the immobilizing anchor having amplifying function of the present example, apparently suggesting that DNA should readily bind to the solid carrier via T4 ligase.

As has been described above, the anchor for immobilizing nucleic acids in accordance with the present invention can concentrate and purify an objective DNA quite specifically in a speedy manner from solutions by immobilizing a desired nucleic acid probe onto an appropriate carrier, and the anchor also can immobilize the DNA through a simple enzymatic reaction. Furthermore, it is evident that the anchor can amplify the binding potency thereof at an extent sufficient for detection by routine DNA cloning technique.

## Claims

1. An anchor for immobilizing nucleic acids, containing a solid carrier, wherein the solid carrier is provided with amplifying groups each having a plurality of branched ends and at a plurality of the branched ends are individually formed binding groups each capable of binding to a functional group arranged at one end of a DNA fragment which is to bind to a preliminarily determined nucleic acid probe.

2. An anchor for immobilizing nucleic acids according to claim 1, wherein each amplifying group is composed of a polymer containing a plurality of binding groups each capable of binding to the functional group within the monomer thereof.

3. An anchor for immobilizing nucleic acids according to claim 1, wherein the amplifying group is composed of a polymer selected from a group consisting of polypeptide compounds, cellulose compounds, polyvinyl alcohol compounds, acrylic compounds, or vinyl compounds.

4. An anchor for immobilizing nucleic acids according to claim 1, wherein the amplifying group is composed of poly-lysine and the DNA fragment binds to an end branched from the NH₂ groups of the poly-lysine.

5. A method for preparing an anchor for immobilizing nucleic acids to form binding groups each capable of binding to a functional group arranged at one end of a DNA fragment which is to bind to a preliminarily determined nucleic acid probe, comprising a first process of activating a solid carrier and a second process of arranging amplifying groups at activated sites of the solid carrier, each amplifying group having a plurality of branched ends to form the binding groups.

6. A method for preparing an anchor for immobilizing nucleic acids according to claim 5, wherein the second process comprises arranging a polymer containing binding groups each capable of binding to the functional group within the monomer thereof.

7. A method for preparing an anchor for immobilizing nucleic acids according to claim 5, wherein the second process comprises arranging as such amplifying group a polymer selected from a group consisting of polypeptide compounds, cellulose compounds, polyvinyl alcohol compounds, acrylic compounds or vinyl compounds.

8. A method for preparing an anchor for immobilizing nucleic acids according to claim 5, wherein the second process comprises arranging poly-lysine as the amplifying groups wherein the binding groups are formed at the ends branched from the NH₂ groups of the poly-lysine.
